# EUROPEAN PATENT SPECIFICATION

(11) **EP 0 562 210 B1**
(45) Date of publication and mention of the grant of the patent: **03.01.1996**
(21) Application number: 92810206.0
(22) Date of filing: 23.03.1992
(51) Int. Cl.: G01N 21/78

(54) **Method for the determination of polyamines**
Verfahren zum Nachweis von Polyaminen
Méthode pour la détermination de polyamines

(43) Date of publication of application: 29.09.1993
(73) Proprietor: FABORGA S.A., Genève (CH)
(72) Inventor: Graf, Anton, Genf (CH)
(74) Representative: Gachnang, Hans Rudolf

(56) References cited:
- DE-A- 3 405 592
- US-A- 4 357 105
- SOVIET PATENTS ABSTRACTS Week 9132, Derwent Publications Ltd., London, GB; AN 91-236546 ; & SU-A-1 594 398
- PATENT ABSTRACTS OF JAPAN vol. 12, no. 42 (P-663)(2889) 6 February 1988 ; & JP-A-62 188 922
- ANALYTICA CHIMICA ACTA, vol. 63, 1 October 1973, pp. 255-261 ; T.M. Florence et al. : "Spectrophotometric determination of p.p.b. levels of long-chain amines in waters and raffinates"
- SOVIET PATENTS ABSTRACTS Week 8937, Derwent Publications Ltd., London, GB; AN 89-269649 ; & SU-A-1 467 466
- ANALYTICAL CHEMISTRY, vol. 47, no. 4, 1 April 1975, pp. 738-741 ; J.P. Rawat et al. : "Sensitive method for spectrophotometric determination of amines"
- ANALYTICA CHIMICA ACTA, vol. 237, 1 September 1990, pp. 391-397 ; R. Stevanato et al. : "Spectrophotometric assay for total polyamines by immobilized amine oxidases"

## Description

This invention relates to a new and useful method for a simple, rapid and sensitive determination of one or more polyamines, such as high molecular weight polyamines, in liquids, especially water. The invention is further related to a new photometer for performing the determination of polyamines.

According to standard literature, see e.g. Römpp's Chemie-Lexikon, column 3273, polyamines are a group of open chain or cyclic organic compounds having two or more primary, secondary or tertiary amino groups in their molecule. These polyamines may have a low, medium, or high molecular weight. The use of polyamines as corrosion inhibitors and as scale inhibitors in industrial plants and installations which employ water for the steam production, heat transfer, or evaporation processes, is increasingly well known. Their effect to inhibit corrosion has been studied, and it has been found that the polyamines form a film comprising an organometallic complex on metal surfaces. This film adheres more tenaciously to surfaces when carboxylic acid polymers such as maleic, vinylic or acrylic polymers or copolymers are also present, see EP-A-0 184 558 which describes a new scale inhibiting method.

The amount of polyamines requested to bring about a desired surface protection are very small and are of the order of about 0.5 to 100 mg/l of water supply. Overdoses as well as underdoses of the added polyamines are undesirable. For this reason, the determination of polyamines in water and other liquids is important. However, the determination of polyamines in the presence of the above mentioned carboxylic polymers is highly problematic, since they interfere with the polyamines during their specific reactions when one tries to determine them by titration. The presence of complexes of such polymers with polyamines has been determined by a general method using sophisticated nuclear magnetic resonance or infrared spectrometry.

Up to now, the precise determination of the level of polyamines in liquid samples is generally conducted by gas chromatography of their fluoroacetyl derivatives, e.g. according to the French CECA-PROCHINOR methodology, which not only uses heavy and complicated apparatuses such as a flow-back coiled glass refrigerator, a flame ionisation column and detector, but also an ultrafine sampling method requiring very small quantities of pure polyamines, namely about 0.2 µl thereof. All these requirements may fit in with scientific research but are not practicable in industry.

Furthermore, a fast determination of polyamines is required for industrial and security reasons in view of a rapid adjustment of corrosion or scale inhibitor introduction into the water circuits or feed immediately after determination, whereas the above described assay methods require a long processing time.

Other, more rapid methods for the determination of polyamines are however known. In one method, the polyamine is complexed with methyl orange, and the intensity of the yellow colour which forms is measured at a wavelength of 430 nm against a reagent blank. Another method comprises addition of disulfine blue, e.g. VN 150, and measuring the absorption curve at a wavelength of 625 nm in a chloroform solution against a standard reagent like HYAMINE 1622 (available from Rohm and Haas).

The international standard No. ISO 2871-1 (May 1989) is based on the titration of an aliquot sodium laurylsulfate standard solution with a solution of a test sample according to the two-phase direct titration method of ISO 2871 standard.

The most serious drawbacks of these titration methods are the necessary use of chloroform as an extraction solvent, since chloroform is highly toxic, and the requirement of using light wavelengths of 430 nm or, respectively, 625 nm which exclude a precise determination of the coloration over a large concentration range which is, as stated above, between about 0.5 and about 100 mg/l.

For these reasons, the use of the above described methods in industrial plants is quite complicated, costly and expensive, time consuming and too much limited in the range of concentrations.

One object of this invention was to find a new and useful method for the rapid, simple and sensitive determination of polyamines in liquids, covering the required concentration range of polyamines, especially water, which does not show the above described drawbacks. The invention aims at providing a method for the determination of polyamines in liquids, especially water, wherein a reagent is added to the polyamine containing liquid which forms a colour by reaction with the polyamine and the colour is detected by means of a photometer at substantially one given wavelength where the colour has an absorption peak.

The apparatus disclosed in US-A-4 357 105, DE-A-3 405 592, and PATENT ABSTRACTS OF JAPAN, vol. 12, no. 44, (P-663) all disclose the use of an LED source, and a filter for measurement on a sample contained in a cuvette by photometric detection.

The invention provides furthermore a new photometer for the determination of polyamines in liquids, especially water, wherein a reagent is added to the polyamine containing liquid which forms a colour by reaction with the polyamine and the colour is detected by means of a photometer at substantially one given wavelength where the colour has an absorption peak, and this photometer is the subject of claim 9. Special embodiments of the method and the photometer are defined in dependent claims.

In the following description of special embodiments of the invention, a particular determination system and photometer are contemplated. However, it should be understood that the method of the invention is not limited to water as the liquid, to a special wavelength of determination, to special light sources (in the LED group) and glass filters, etc.

In the Figures:
- Fig. 1 shows the absorption curve, intensity (ordinate) versus absorption, in terms of wavelength in nm (abscissa) of the colour of a reaction product of a polyamine with Bengalrosa B.A. lake;
- Fig. 2 represents the spectral distribution (intensity versus wavelength) of the light emitted by a standard green LED;
- Fig. 3 shows the transmission range (intensity versus wavelength in nm) of a plain green glass filter;
- Fig. 4 shows the transmission curve (intensity versus wavelength in nm) of the LED light filtered through the glass filter of Fig. 3;
- Fig 5 shows in purely schematical representation a photometer of this invention, and
- Fig 6 shows the relation between the degree of transmission (ordinate) and the polyamine concentration in mg/l (abscissa).

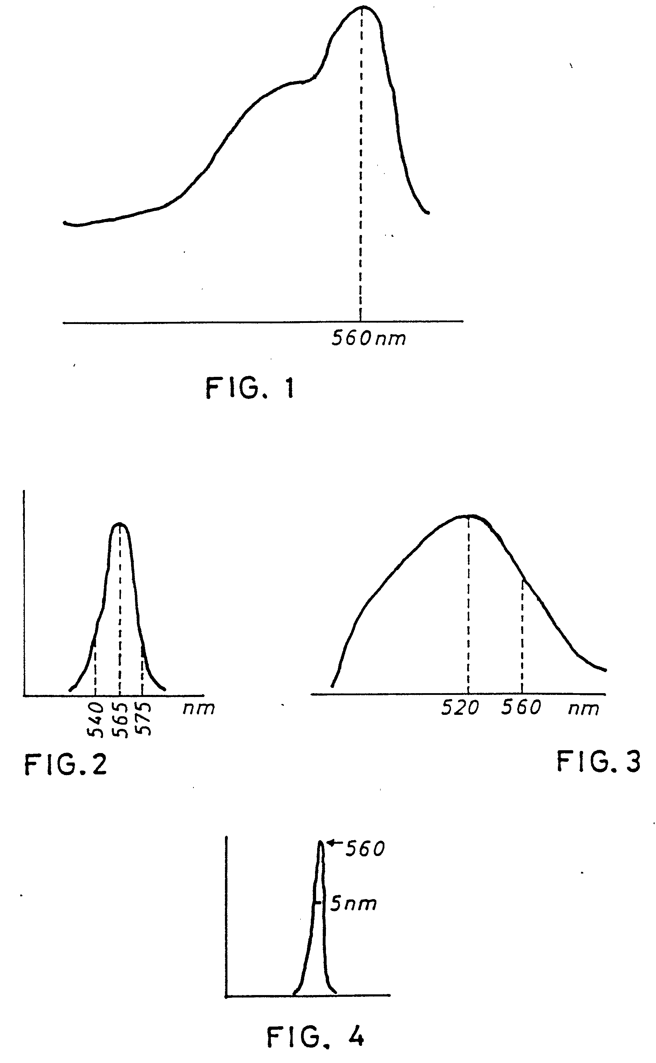

The preferred colour forming reaction in the method of the invention is based on the reaction of the polyamines to be assayed with sodium 3,4,5,6-tetrachloro-2',4',5',7'-tetraiodofluorescein, commonly known as Bengalrosa B.A. lake which yields a stable reddish colour soluble in diluted acetic acid. The absorption maximum of this colour is situated at exactly 560 nm, see Fig. 1. This colour reaction is quite specific for polyamines. If the use of this colour reaction for the calibration of transmission against polyamine concentration is desired, the wavelength of the light generated by the photometer has to be exactly 560 nm with an allowable bandwidth of 5 nm. Only under this condition, the photometric measurements will be sensitive at the lower desired concentration level of about 0.5 mg of polyamine per liter. Known UV/VIS photometers would require the use of costly interference filters of the above mentioned wavelength, and a photometer specially designed for the use of such filters. The whole installation, even in the case of grating or prism photometers, will become expensive because larger path-length cuvettes have to be accomodated to increase the sensitivity of the colour determination.

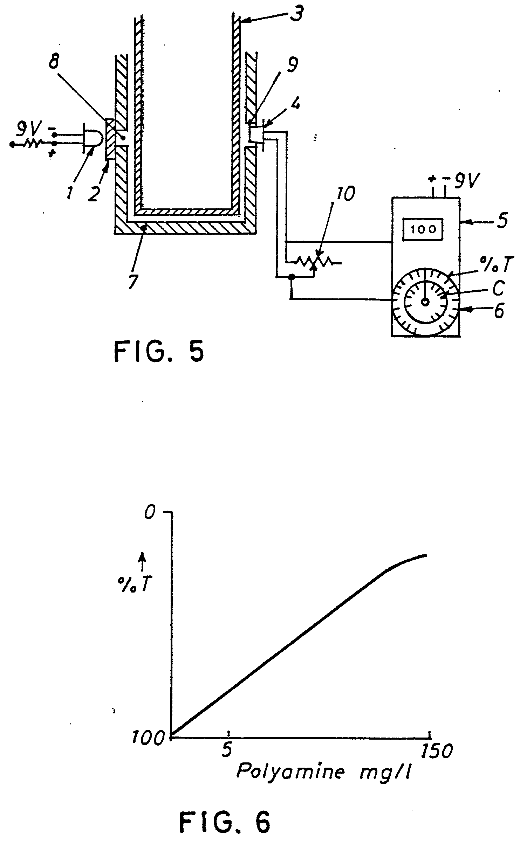

All these difficulties could surprisingly be eliminated by the photometer of this invention. The sole light source of this simple photometer is a conventional green light emitting diode (LED). Such a LED has however a rather broad spectral distribution shown in Fig. 2. It covers, in addition to green, some portions of orange and yellow light in that the emission range is from 540 to 575 nm. If one would use this light source alone for the assays, the transmission results would be low making the determination insensitive for low polyamine concentrations. The present invention contemplates the arrangement of a cheap glass filter having a (broad) transmission range of from about 540 to about 560 nm, see Fig. 3, in front of the green LED. Most surprisingly, it has been found that the light of the LED having passed through the glass filter has now a narrow-width, sharp- peak wavelength distribution of 560 nm, see Fig. 4, and is to be considered as substantially monochromatic. The effect of the glass filter is due to a general weakening of the spurious wavelengths on both sides of the peak. Fig 5 now shows the schematical construction of a photometer of this invention.

The photometer comprises a housing 7 in which a glass cuvette, having a diameter of, e.g., 3 cm is inserted. The housing has two facing lateral openings 8 and 9 in lateral walls. Opening 8 is covered with a glass filter 2 having a transmission range of from 540 to 560 nm, see Fig. 3. A green LED 1, connected via an appropriate resistor to a dc current source of, e.g., 9 V, is mounted behind the glass filter in such a way that the light coming from the LED passes by the glass filter 2 through the opening 8, then through the cuvette 3 and the opening 9 to impinge upon a conventional green sensitive photodiode 4 mounted in the opening 9. The current from the photodiode 4 is applied via a potentiometer 10 to a millivoltmeter 5 whose range is set to 0-100 mV. This millivoltmeter is further equipped with a manually operated conversion scale 6 to convert the percental transmission given by the millivolt reading, to the concentration of polyamines in the sample within the cuvette 3.

### EXAMPLE

Colorimetric determination of polyamines in the treatment of boiler water.

The following reagents were used:

| Reagent 1 | |
|---|---|
| Bengalrosa | 500 mg |
| Ethanol | 400 ml |
| Quartz distilled water | 600 ml. |

| Reagent 2 | |
|---|---|
| Acetic acid, p.a. | 500 ml |
| Quartz distilled water | 500 ml. |

25 ml of polyamine containing boiler water are filled in a round cuvette having a diameter of 3 cm. 1 ml of reagent 1 and 2 ml of reagent 2 are added. In another cuvette, 25 ml quartz distilled water are treated in the same manner and used as a blank to adjust the photometric transmission to 100%. The millivoltmeter reading obtained when the sample cuvette is inserted into the photometer, is recorded and converted into polyamine concentration by using a calibration scale or curve obtained with a series of standards containing known amounts of polyamines. The base of this calibration is shown in Fig. 6 wherein the polyamine concentration is brought into correlation with the observed transmission of the photometer cell in %.

As it can be seen from Fig. 6, the photometer of this invention covers the whole desired polyamine concentration range of from about 0.5 to 100 mg/l in the water samples.

The invention can further be completed. Thus, it is possible to adapt the determination method to a continuous measurement of the polyamine concentration in, e.g., boiler supply water. In this case, the cuvette 3 in Fig. 5 is replaced by a flow-through cell, and the reagents are added by means of dosing pumps. Further modifications and additions to the subjects of this invention can be imagined and are protected in the scope of the appended claims.

## Claims

1. A method for the determination of polyamines in liquids, wherein a reagent is added to the polyamine containing liquid, said reagent forming a colour by reaction with the polyamine and this colour is measured by light at substantially one given wavelength where the colour has an absorption peak, by means of a photometer comprising a light emitting diode (LED) and a light filter placed in front of the LED, wherein the liquid is treated with a reagent forming with said polyamine a colour which has a sharp absorption peak, said LED emits light at both wavelength sides of said peak, said filter is a glass filter which has a transmission maximum which is broad relative to the LED spectral distribution and the absorption peak, the maximum being at a shorter wavelength than said absorption peak, and the concentration of the polyamine in said liquid is evaluated in response to the intensity of the nearly monochromatic light transmitted through the liquid in said photometer.

2. The method of claim 1, characterized by the fact that said liquid is water.

3. The method of claim 2, characterized by the fact that said water is boiler supply water or industrial circulating water.

4. The method of any one of claims 1 to 3, characterized by the fact that said reagent is sodium 3,4,5,6-tetrachloro-2',4',5',7'-tetraiodofluorescein, and the reaction of this reagent with one or more polyamines forms a stable colour in the presence of a weak acid, said colour having an absorption maximum at about 560 nm.

5. The method of claims 1 and 4, characterized by the fact that a green LED is used emitting light of wavelengths comprised between 540 and 575 nm.

6. The method of claims 4 or 5, characterized by the fact that a glass filter is used having a transmission maximum at about 520 nm and a transmission range at both wavelength sides of that maximum.

7. The method of any one of the preceding claims, characterized by the fact that a polyamine concentration is continuously determined by using a flow-through cuvette in said photometer and adding colour forming reagents continuously to said flow-through cuvette.

8. The method of any one of the preceding claims, characterized by the fact that the photometer readings are made on a millivoltmeter, and that the measured millivolts are automatically converted into values of polyamine concentration.

9. A photometer for the determination of polyamines in liquids, especially water, wherein a colour formed by a reaction with the polyamine is detected at substantially one given wavelength where the colour has an absorption peak, said photometer comprising a LED (1) as a light source, a filter (2) placed in front of the LED, a cuvette (3) for receiving the polyamine containing liquid sample, and a light detector device (5) comprising a photodiode (4), wherein the filter (2) is a glass filter having a transmission maximum which is broad relative to the LED spectral distribution and the absorption peak, the maximum being at a shorter wavelength than the LED spectral distribution maximum and said absorption peak and thus being capable of reducing the wavelength range emitted by the LED (1) to a sharp band, and the light detector device (5) is adapted to convert the light signal coming from the LED (1) through the filter (2) and the cuvette (3) into polyamine concentration.

10. The photometer of claim 9, characterized by the fact that it is adapted to receive aqueous samples wherein the polyamine concentration is from about 0.5 to about 100 mg/l.

11. The photometer of claim 9 or 10, characterized by the fact that the LED (1) is a LED emitting green light in a broad wavelength range and a peak at a wavelength of about 565 nm, and a glass filter is used whose transmission has a broad peak covering 540 to 560 nm.

12. The photometer of any one of claims 9 to 11, characterized by the fact that that the cuvette is a flow-through cell adapted to receive continuously colour forming reagents by means of dosing pumps, the photometer thus being arranged for the continuous determination of polyamines in liquids, especially boiler supply water.

## Patentansprüche

1. Verfahren zur Bestimmung von Polyaminen in Flüssigkeiten, bei dem zur polyaminhaltigen Flüssigkeit ein Reagenz zugegeben wird, welches durch Reaktion mit dem Polyamin eine Färbung bildet und diese Färbung mittels Licht von im wesentlichen einer gegebenen Wellenlänge, bei der die Färbung einen Absorptionspeak aufweist, in einem Photometer gemessen wird, das eine Lumineszenzdiode (LED) und ein vor der LED angeordnetes Lichtfilter aufweist, wobei die Flüssigkeit mit einem Reagenz behandelt wird, das mit dem genannten Polyamin eine Färbung bildet, welche einen scharfen Absorptionspeak aufweist, die genannte LED Licht mit Wellenlängen beiderseits des genannten Peaks emittiert, das genannte Filter ein Glasfilter ist, welches ein Durchlässigkeits-Maximum besitzt, das im Vergleich zur Spektralverteilung der LED und zum Absorptionspeak breit ist und dieses Maximum bei einer kürzeren Wellenlänge als derjenigen des genannten Absorptionspeaks liegt, und wobei die Konzentration des Polyamins in der genannten Flüssigkeit in Abhängigkeit von der Intensität des nahezu monochromatischen Lichtes bestimmt wird, das durch die Flüssigkeit im genannten Photometer hindurchtritt.

2. Verfahren nach Anspruch 1, dadurch gekennzeichnet, dass die genannte Flüssigkeit Wasser ist.

3. Verfahren nach Anspruch 2, dadurch gekennzeichnet, dass das genannte Wasser Kesselspeisewasser oder im Kreislauf geführtes Brauchwasser ist.

4. Verfahren nach einem der Ansprüche 1 bis 3, dadurch gekennzeichnet, dass das genannte Reagenz Natrium-3,4,5,6-tetrachlor-2',4',5',7'-tetraiodfluorescein ist, und dass die Umsetzung dieses Reagenz mit einem oder mehreren Polyaminen in Gegenwart einer schwachen Säure eine stabile Färbung hervorruft, welche ein Absorptionsmaximum bei etwa 560 nm besitzt.

5. Verfahren nach den Ansprüchen 1 und 4, dadurch gekennzeichnet, dass eine grüne LED verwendet wird, welche Licht von Wellenlängen im Bereich von 540 bis 575 nm emittiert.

6. Verfahren nach Anspruch 4 oder 5, dadurch gekennzeichnet, dass ein Glasfilter verwendet wird, das ein Durchlässigkeits-Maximum bei etwa 520 nm und einen Durchlassbereich bei Wellenlängen beiderseits dieses Maximum aufweist.

7. Verfahren nach einem der vorstehenden Ansprüche, dadurch gekennzeichnet, dass eine Polyaminkonzentration laufend bestimmt wird, indem im genannten Photometer eine Durchflussküvette verwendet wird und Farbbildungs-Reagenzien laufend in die genannte Durchflussküvette eingespeist werden.

8. Verfahren nach einem der vorstehenden Ansprüche, dadurch gekennzeichnet, dass die Photometeranzeige an einem Millivoltmeter abgelesen wird, und dass die gemessenen Millivolt automatisch in Werte der Polyaminkonzentration umgewandelt werden.

9. Photometer zur Bestimmung von Polyaminen in Flüssigkeiten, insbesondere Wasser, worin eine durch eine Reaktion mit dem Polyamin gebildete Färbung bei im wesentlichen einer gegebenen Wellenlänge, bei der die Färbung einen Absorptionspeak aufweist, bestimmt wird, und wobei das Photometer als Lichtquelle eine LED (1), ein vor der LED angeordnetes Filter (2), eine Küvette (3) zur Aufnahme der polyaminhaltigen Flüssigkeitsprobe und eine eine Photodiode (4) enthaltende Lichtdetektor-Anordnung (5) aufweist, wobei das Filter (2) ein Glasfilter ist, welches ein Durchlässigkeits-Maximum besitzt, das gegenüber der Spektralverteilung der LED und dem Absorptionspeak breit ist und dieses Maximum bei einer kürzeren Wellenlänge als das Maximum der Spektralverteilung der LED und derjenigen des genanntem Absorptionspeaks liegt, und welches daher den von der LED (1) abgestrahlten Wellenlängenbereich auf eine scharfe Bande reduziert, und wobei die Lichtdetektor-Anordnung (5) zur Umwandlung des von der LED (1) durch das Filter (2) und die Küvette (3) kommende Lichtsignal in Polyaminkonzentration eingerichtet ist.

10. Photometer nach Anspruch 9, dadurch gekennzeichnet, dass es zur Aufnahme von wässrigen Proben eingerichtet ist, in denen die Polyaminkonzentration zwischen etwa 0,5 und etwa 100 mg/l beträgt.

11. Photometer nach Anspruch 9 oder 10, dadurch gekennzeichnet, dass die LED (1) eine solche ist, die grünes Licht in einem breiten Wellenlängenbereich mit einem Peak bei einer Wellenlänge von etwa 565 nm emittiert, und dass ein Glasfilter verwendet ist, dessen Durchlässigkeit einen breiten, 540 bis 560 nm überdeckenden Bereich aufweist.

12. Photometer nach einem der Ansprüche 9 bis 11, dadurch gekennzeichnet, dass die Küvette eine Durchflusszelle ist, die zur kontinuierlichen Zufuhr von farbbildenden Reagenzien mittels Dosierpumpen ausgebildet ist, wobei das Photometer demgemäss zur kontinuierlichen Bestimmung von Polyaminen in Flüssigkeiten, insbesondere Kesselspeisewasser, eingerichtet ist.

## Revendications

1. Procédé pour la détermination de polyamines dans des liquides, procédé dans lequel un réactif est ajouté au liquide contenant la polyamine, le dit réactif formant une couleur par réaction avec la polyamine et cette couleur étant mesurée par la lumière à substantiellement une longueur d'onde où la couleur présente une pointe d'absorption, au moyen d'un photomètre comprenant une diode à luminescence (DEL) et un filtre de lumière placé devant la DEL, et dans lequel le liquide est traité avec un réactif formant avec la dite polyamine une couleur présentant une pointe nette d'absorption, la dite DEL émettant de la lumière de part et d'autre de la longueur d'onde de la dite pointe, le dit filtre étant un filtre en verre présentant un maximum de transmission large par rapport à la distribution spectrale de la DEL et à la pointe d'absorption, ce maximum étant situé à une longueur d'onde plus courte que la dite pointe d'absorption, et la concentration de la polyamine dans le dit liquide est évaluée en fonction de l'intensité de la lumière presque monochromatique transmise àtravers le liquide dans le dit photomètre.

2. Procédé selon la revendication 1, caractérisé par le fait que le dit liquide est l'eau.

3. Procédé selon la revendication 2, caractérisé par le fait que la dite eau est de l'eau alimentaire de chaudière ou de l'eau industrielle en circulation.

4. Procédé selon l'une quelconque des revendications 1 à 3, caractérisé par le fait que le dit réactif est la 3,4, 5,6-tétrachloro-2',4',5',7'-tétraiodofluorescéine de sodium, et que la réaction de ce réactif avec une ou plusieurs polyamines forme une couleur stable en présence d'un acide faible, la dite couleur ayant un maximum d'absorption à environ 560 nm.

5. Procédé selon les revendications 1 et 4, caractérisé par le fait qu'une DEL verte est utilisée émettant de la lumière des longueurs d'onde comprises entre 540 et 575 nm.

6. Procédé selon la revendication 4 ou 5, caractérisé par le fait qu'un filtre en verre est utilisé ayant un maximum de transmission à environ 520 nm et une gamme de transmission aux longueurs d'onde de part et d'autre de ce maximum.

7. Procédé selon l'une quelconque des revendications précédentes, caractérisé par le fait que la concentration d'une polyamine est déterminée de manière continue en utilisant une cuvette de circulation dans le dit photomètre, et en ajoutant les réactifs formateurs de couleur en continu dans la dite cuvette de circulation.

8. Procédé selon l'une quelconques des revendications précédentes, caractérisé par le fait que les lectures du photomètre sont effectuées sur un millivoltmètre, et que les millivolts mesurés sont automatiquement convertis en valeurs de concentration de polyamine.

9. Photomètre pour la détermination de polyamines dans des liquides, en particulier dans l'eau, photomètre dans lequel une couleur formée par une réaction avec la polyamine est détectée à substantiellement une longueur d'onde où la couleur présente une pointe d'absorption, le dit photomètre comprenant une DEL (1) comme source de lumière, un filtre (2) placé devant la DEL, une cuvette (3) pour recevoir l'échantillon de liquide contenant la polyamine, et un dispositif détecteur de lumière (5) comportant une photodiode (4), le filtre (2) étant un filtre en verre présentant un maximum de transmission large par rapport à la distribution spectrale de la DEL et à la pointe d'absorption, ce maximum étant situé à une longueur d'onde plus courte que le maximum de la distribution spectrale de la DEL et que la dite pointe d'absorption, le filtre étant alors capable de réduire la gamme de longueurs d'onde émise par la DEL (1) à une bande nette, le dispositif détecteur de lumière (5) étant adapté pour convertir le signal lumineux provenant de la DEL (1) à travers le filtre (2) et la cuvette (3), en concentration de polyamine.

10. Photomètre selon la revendication 9, caractérisé par le fait qu'il est adapté pour recevoir des échantillons aqueux dans lesquels la concentration de polyamine est comprise entre environ 0,5 et environ 100 mg/l.

11. Photomètre selon la revendication 9 ou 10, caractérisé par le fait que la DEL (1) est une DEL émettant de la lumière verte dans une large gamme de longueurs d'onde et présentant une pointe située à une longueur d'onde d'environ 565 nm, et qu'un filtre en verre est utilisé dont la transmission présente une large pointe couvrant la gamme de 540 à 560 nm.

12. Photomètre selon l'une quelconque des revendications 9 à11, caractérisé par le fait que la cuvette est une cuve de circulation agencée pour recevoir en continu des réactifs formateurs de couleur amenés au moyen de pompes de dosage, le photomètre étant donc agencé pour la détermination en continu de polyamines dans des liquides, en particulier dans l'eau d'alimentation de chaudière.
